# FASCICULE DE BREVET EUROPEEN

(11) **EP 0 841 853 B1**
(45) Date de publication et mention de la délivrance du brevet: **22.10.2003**
(21) Numéro de dépôt: 96926445.6
(22) Date de dépôt: 23.07.1996
(51) Int. Cl.: A01N 57/12

(54) **COMPOSITION FONGICIDE SYNERGIQUE COMPRENANT UN COMPOSE ANALOGUE DE LA STROBILURINE**
EINE STROBILURINANALOGVERBINDUNG ENTHALTENDE SYNERGISTISCHEFUNGIZIDE ZUSAMMENSETZUNG
SYNERGISTIC FUNGICIDAL COMPOSITION INCLUDING A STROBILURINE ANALOGUE COMPOUND

(30) Priorité: 24.07.1995 FR 9509183
(43) Date de publication de la demande: 20.05.1998
(73) Titulaire: Bayer CropScience S.A., 69009 Lyon (FR)
(72) Inventeur: DUVERT, Patrice, F-69004 Lyon (FR)
(86) Numéro de dépôt international: FR9601155
(87) Numéro de publication internationale: WO97003563

(56) Documents cités:
- WO-A-95/15083
- RESEARCH DISCLOSURE, vol. 338, no. 33893, Juin 1992, HAVANT GB, pages 506-510, XP000315713 "Mixtures of fungicides and insecticides."
- RESEARCH DISCLOSURE, vol. 348, no. 34874, Avril 1993, HAVANT GB, pages 267-268, XP000304224 "Mixtures of fungicides and herbicides."

## Description

La présente invention a pour objet une composition fongicide synergique comprenant un composé analogue de la strobilurine et un procédé mettant en oeuvre ladite composition et destiné à protéger, à titre curatif ou préventif, les cultures contre les attaques fongiques.

On connaît, notamment par les demandes de brevet européen EP 253213 ou 398692 ou par la demande internationale WO 9208703, des composés analogue de la strobilurine à action fongicide, permettant de prévenir la croissance et le développement de champignons phytopathogènes susceptibles d'attaquer les cultures.

WO 95/15083 décrit un mélange fongicide d'analogues de la strobilurine avec un second fongicide, par example du fosétyl-Al.

Il est cependant toujours désirable d'améliorer le spectre d'activité et l'efficacité de tels composés à action fongicide, ou de les renforcer en les associant à d'autres molécules afin d'obtenir un produit plus performant (association avec un fongicide systémique, ces fongicides étant plutôt des molécules de type "contact") ou encore de prévenir l'apparition de souches fongiques résistantes à ces nouveaux fongicides.

Il est également très souhaitable de disposer de produits fongicides bénéficiant d'une persistance d'action améliorée, de nature à espacer dans le temps le nombre de traitements phytosanitaires nécessaires au bon contrôle des parasites.

Il est dans tous les cas particulièrement avantageux de pouvoir diminuer la quantité de produits chimiques épandus dans l'environnement, tout en assurant une protection performante des cultures contre les attaques fongiques.

Il a maintenant été trouvé qu'un (ou plusieurs) des objectifs précédents pouvait être atteint grâce à la composition fongicide selon la présente invention.

La présente invention a donc pour objet en premier lieu une composition fongicide synergique comprenant un composé A qui est le méthyl-(E)'-2-{2-[6-(2-cyanophenoxy)pyrimidin--4-yloxy]phenyl}-3-methoxyacrylate
et au moins un composé fongicide B choisi dans le groupe comprenant les dérivés de l'acide phosphoreux comme les phosphites métalliques tel que le fosétyl-Al, et l'acide phosphoreux lui-même et ses sels alcalins ou alcalino-terreux.

La composition fongicide selon l'invention comprend avantageusement les composants A et B dans un rapport en poids A/B, compris entre 0,004 et 1, de préférence entre 0,0125 et 0,4.

Il est bien entendu que ladite composition fongicide peut renfermer un seul composé B ou plus d'un tel composé, par exemple 1, 2 ou 3 composés B selon l'utilisation à laquelle elle est destinée.

Parmi les significations plus spécialement préférées du composé B définies ci-dessus, on préfère encore le fosétyl Al. De manière parfaitement inattendue, la composition selon l'invention améliore alors de façon notable l'action des matières actives prises séparément pour un nombre de champignons particulièrement nuisibles pour les cultures, comme en particulier la vigne ou les solanées. Cette amélioration se traduit notamment par une diminution des doses de chacun des constituants, ce qui est particulièrement avantageux pour l'utilisateur et l'environnement. Le produit fongicide présente ainsi des propriétés synergiques attestées par l'application de la méthode de Tammes, "Isoboles, a graphic representation of synergism in pesticides" Netherlands Journal of Plant Pathology, 70(1964), p. 73-80.

De manière préférée, lorsque le composant B est le fosétyl-Al, le rapport A/B est compris entre 0,01 et 1, de préférence entre 0,033 et 0,4 pour l'ensemble des cultures envisagées.

Dans le cas particulier du gazon, le rapport A/B sera compris entre 0,004 et 0,4, de préférence entre 0,0125 et 0,1.

Les structures correspondant aux noms communs des matières actives fongicides figurant dans la définition de B sont indiquées dans l'un au moins des 2 ouvrages suivants:
- "The pesticide manual" édité par Clive TOMLIN et publié par le British Crop Protection Council, 10ème édition ;
- l'Index phytosanitaire 1994, édité par l'Association de Coordination Technique Agricole, 30ème édition.

Le méthyl-(E)-2-{2-[6-(2-cyanophenoxy)pyrimidin-4-yloxy]phenyl}-3-methoxyacrylate (ou ICIA5504) est décrit dans la demande internationale WO 9208703. Par ailleurs, ICIA5504 est répertorié dans l'ouvrage "The pesticide manual" précité.

La composition fongicide selon l'invention comprend, comme matière active, un composé A et au moins un composé B en mélange avec les supports solides ou liquides, acceptables en agriculture et/ou les agents tensio-actifs également acceptables en agriculture. En particulier sont utilisables les supports inertes et usuels et les agents tensio-actifs usuels. Ces compositions recouvrent non seulement les compositions prêtes à être appliquées sur la culture à traiter au moyen d'un dispositif adapté, tel qu'un dispositif de pulvérisation, mais également les compositions concentrées commerciales qui doivent être diluées avant application sur la culture. On désigne par matière active la combinaison d'un composé A avec au moins un composé B.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... Plus généralement les composés A et B peuvent être combinés à tous les additifs solides ou liquides correspondant aux techniques habituelles de la mise en formulation.

D'une façon générale, les compositions selon l'invention contiennent habituellement de 0,05 à 95 % (en poids) de matière active, un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle la matière active est combinée pour faciliter son application sur les parties aériennes de la plante. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, notamment le butanol etc...).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque la matière active et/ou le support inerte ne sont pas solubles dans l'eau et que l'agent vecteur de l'application est l'eau.

Ainsi donc, les compositions à usage agricole selon l'invention peuvent contenir la matière active dans de très larges limites, allant de 0,05 % à 95 % (en poids). Leur teneur en agent tensio-actif est avantageusement comprise entre 5 % et 40 % en poids. Sauf indication contraire les pourcentages donnés dans cette description, incluant les revendications, sont en poids.

Ces compositions selon l'invention sont elles-mêmes sous des formes assez diverses, solides ou liquides.

Comme formes de compositions solides, on peut citer les poudres pour poudrage (à teneur en matière active pouvant aller jusqu'à 100 %) et les granulés, notamment ceux obtenus par extrusion, par compactage, par imprégnation d'un support granulé, par granulation à partir d'une poudre (la teneur en matière active dans ces granulés étant entre 0,5 et 80 % pour ces derniers cas), les comprimés ou tablettes effervescents.

La composition fongicide selon l'invention peut encore être utilisée sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les émulsions, les suspensions concentrées, les aérosols, les poudres mouillables (ou poudre à pulvériser), les pâtes, les gels.

Les suspensions concentrées, applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

### Exemple SC 1 :

- matière active 500 g
- phosphate de tristyrylphénol polyéthoxylé 50 g
- alkylphénol polyéthoxylé 50 g
- polycarboxylate de sodium 20 g
- éthylène glycol 50 g
- huile organopolysiloxanique (antimousse) 1 g
- polysaccharide 1,5 g
- eau 316,5 g

Les poudres mouillables (ou poudre à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0,1 à 10 % d'un ou plusieurs stabilisants et/ou autres additifs, comme des agents de pénétration, des adhésifs, ou des agents antimottants, colorants, etc...

Pour obtenir les poudres à pulvériser ou poudres mouillables, on mélange intimement les matières actives dans les mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables trés avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

### Exemple PM 1

- matière active 50%
- alcool gras éthoxylé (agent mouillant) 2,5%
- phényléthylphénol éthoxylé (agent dispersant) 5%
- craie (support inerte) 42,5%

### Exemple PM 2 :

- matière active 10%
- alcool synthétique oxo de type ramifié, en C13 éthoxylé par 8 à 10 oxyde d'éthylène (agent mouillant) 0,75%
- lignosulfonate de calcium neutre (agent dispersant) 12%
- carbonate de calcium (charge inerte) q.s.p. 100 %

### Exemple PM 3 :

Cette poudre mouillable contient les mêmes ingrédients que dans l'exemple précédent, dans les proportions ci-après :
- matière active 75%
- agent mouillant 1,50%
- agent dispersant 8%
- carbonate de calcium (charge inerte) q.s.p. 100%

### Exemple PM 4 :

- matière active 90%
- alcool gras éthoxylé (agent mouillant) 4%
- phényléthylphénol éthoxylé (agent dispersant) 6%

### Exemple PM 5 :

- matière active 50%
- mélange de tensio-actifs anioniques et non ioniques (agent mouillant) 2,5%
- lignosulfonate de sodium (agent dispersant) 5%
- argile kaolinique (support inerte) 42,5%

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les compositions fongicides selon l'invention peuvent être formulées sous la forme de granulés dispersibles dans l'eau également compris dans le cadre de l'invention.

Ces granulés dispersibles, de densité apparente généralement comprise entre environ 0,3 et 0,6 ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus. On peut encore utiliser des granulés obtenus comme précédemment puis imprégnés avec une composition contenant la matière active.

De préférence, il est obtenu par extrusion, en opérant comme indiqué dans les exemples ci-après.

### Exemple GD1 : Granulés dispersibles

Dans un mélangeur, on mélange 90 % en poids de matière active et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

### Exemple GD2 : Granulés dispersibles

Dans un mélangeur, on mélange les constituants suivants :
- matière active 75%
- agent mouillant (alkylnaphtalène sulfonate de sodium) 2%
- agent dispersant (polynaphtalène sulfonate de sodium) 8%
- charge inerte insoluble dans l'eau (kaolin) 15%

Ce mélange est granulé en lit fluide, en présenoe d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

En ce qui concerne les compositions adaptées au stockage et au transport, elles contiennent plus avantageusement de 0,5 à 95 % (en poids) de matière active.

L'invention a pour autre objet un procédé de lutte, à titre curatif ou préventif, contre les champignons phytopathogènes par exemple des cultures ou du gazon, caractérisé en ce que l'on applique sur les parties aériennes des végétaux une quantité efficace et non phytotoxique d'une combinaison d'un composé A et au moins un composé B, par exemple dans une composition fongicide selon l'invention.

Les champignons phytopathogènes des cultures qui peuvent être combattus par ce procédé sont notamment ceux :
- du groupe des oomycètes :
   - du genre *Phytophthora* tel que *Phytophthora infestans* (mildiou des solanées, notamment de la pomme de terre ou de la tomate), *Phytophthora citrophthora, Phytophthora capsici, Phytophthora cactorum, Phytophthora palmivora, Phytophthora cinnamoni, Phytophthora megasperma, Phytophthora parasitica*,
   - de la famille des Péronosporacées, notamment *Plasmopara viticola* ( mildiou de la vigne), *Plasmopara halstedei* (mildiou du tournesol), *Pseudoperonospora sp* (notamment mildiou des cucurbitacees et du houblon), *Bremia lactucae* (mildiou de la laitue), *Peronospora tabacinae* (mildiou du tabac),
- du groupe des adélomycètes :
   - du genre *Alternaria*, par exemple *Alternaria solani* (alternariose des solanées, et notamment de la tomate et des pommes de terre ),
   - du genre *Guignardia*, notamment *Guignardia bidwellii* (black rot de la vigne),
   - du groupe des *Oïdiums*, par exemple oïdium de la vigne (*Uncinula necator*) ; oïdium des cultures légumières, par exemple *Erysiphe polygoni* (oidium des crucifères) ; *Leveillula taurica*, *Erysiphe cichoracearum, Sphaerotheca fuligena*; (oïdium des cucurbitacées, des composées, de la tomate) ; *Erysiphe communis* (oïdium de la betterave et du chou) ; *Erysiphe pisi* (oïdium du pois, de la luzerne) ; *Erysiphe polyphaga* (oïdium du haricot et du concombre) ; *Erysiphe umbelliferarum* (oïdium des ombellifères, notamment de la carotte) ; *Sphaerotheca humuli* (oïdium du houblon) ; *Erysiphe graminis* (oïdium des céréales).
   - du genre *Septoria*, *par* exemple *Septoria nodorum* ou *Septoria tritici* (septoriose des céréales) ;
- du groupe des Basidiomycètes :
   - du genre *Puccinia,* par exemple *Puccinia recondita* ou *striiformis* (rouilles du blé).

Un classement fait non plus par champignons visés mais par cultures cibles peut être illustré comme ci-dessous :
- vigne: oïdium (*Uncinula necator*), mildiou (*Plasmopara viticola*), excoriose (*Phomopsis viticola*) et black-rot (*Guignardia bidwellii*),
- solanées: mildiou (*Phytophthora infestans*), alternariose (*Alternaria solani*) et pourriture (*Botrytis cinerea*),
- cultures légumières: mildious (*Peronospora* sp., *Bremia lactucae, Pseudoperonospora* sp., alternariose (*Alternaria* sp.), sclérotiniose (*Sclerotinia* sp.), pourriture (*Botrytis cinerea*), oïdium (*Erysiphe* sp.; *Sphaerotheca fuliginea*)
- arboriculture: tavelure (*Venturia inaequalis*), oïdium (*Podosphaera leucotricha*) et moniliose (*Monilia fructigena*),
- agrumes: tavelure (*Elsinoe fawcetti*), mélanose (*Phomopsis citri*) et maladies *à Phytophthora* sp.,
- banane: cercosporiose (*Mycosphaerella figiensis*),
- gazon: rouille, oïdium, helminthosporiose, maladies telluriques (*Microdochium nivale, Pythium* sp., *Rhizoctonia solani*,...).

La composition fongicide objet de l'invention est appliquée au moyen de différents procédés de traitement tels que :
- la pulvérisation sur les parties aériennes des cultures à traiter d'un liquide comprenant ladite composition,
- le poudrage, l'incorporation au sol de granulés ou de poudres, l'arrosage, l'injection dans les arbres ou le badigeonnage.

La pulvérisation d'un liquide sur les parties aériennes des cultures à traiter est le procédé de traitement préféré.

Par "quantité efficace et non phytotoxique", on entend une quantité de composition selon l'invention suffisante pour permettre le contrôle ou la destruction des champignons présents ou susceptibles d'apparaître sur les cultures, et n'entraînant pour lesdites cultures aucun symptôme notable de phytotoxicité. Une telle quantité est susceptible de varier dans de larges limites selon le champignon à combattre, le type de culture, les conditions climatiques, et les composés compris dans la composition fongicide selon l'invention. Cette quantité peut être déterminée par des essais systématiques au champ, à la portée de l'homme du métier.

Les doses d'emploi lors de la mise en oeuvre du procédé selon l'invention seront généralement alors :
sur vigne, cultures légumières, solanées, banane, arboriculture, agrumes:
   500 à 5000g de composé B, par ex. fosétyl-Al + 50 à 500 g/ha de composé A et plus précisément 1000 à 3000g + 100 à 400 g/ha, soit une dose totale de composition selon l'invention comprise entre 550 et 5500 g/ha, de préférence entre 1100 et 3400 g/ha.
sur gazon:
   5000 à 25000g de composé B, par ex. fosétyl-Al + 100 à 2000 g/ha de composé A et plus précisément 10000 à 20000g + 250 à 1000 g/ha, soit une dose totale de composition selon l'invention comprise entre 5100 et 27000 g/ha, de préférence entre 10250 et 21000 g/ha.

Dans les figures jointes au présent texte, la dose de chaque matière active prise isolément, requise pour le contrôle du champignon phytopathogène au niveau indiqué, est comparée avec celle des 2 matières actives prises en mélange. La dose efficace de chaque matière active prise isolément est indiquée sur l'axe des abscisses et des ordonnées et une ligne droite est tracée coupant ces 2 axes et reliant ces 2 doses. Lorsqu'une matière active prise isolément n'est pas efficace la ligne droite est parallèle à l'axe des coordonnées qui indique les doses de cette matière active. En ce qui concerne les 2 matières actives prises en mélange, la dose du mélange à un ratio donné est indiquée par un point

### Exemple 1 : Essai in vivo de l'association de ICIA5504 avec le fosétyl-Al sur Plasmopara viticola (mildiou de la vigne) par traitement préventif 72 heures avant contamination.

On prépare une suspension comprenant les composés A et B dans un mélange liquide constitué d'un agent tensioactif (oléate de dérivé polyoxyéthyléné du sorbitan) et d'eau.

Le composant B est le fosétyl-Al ; le rapport A/B est 0,05, 0,07 et 0,1 (B/A = 20, 15 et 10).

Des boutures de vigne (Vitis vinifera), variété Chardonnay, sont cultivées dans des godets. Lorsque ces plants sont âgés de 2 mois (stade 8 à 10 feuilles, hauteur de 10 à 15 cm ), ils sont traités par pulvérisation au moyen de la suspension ci-dessus.

Des plants utilisés comme témoins sont traités par une suspension similaire mais ne contenant pas de matière active ("blanc de formulation").

Après 72 heures, on contamine chaque plant par pulvérisation d'une suspension aqueuse de spores de *Plasmopara viticola* obtenue à partir de feuilles sporulées contaminées 7 jours auparavant. Ces spores sont mises en suspension à raison de 100 000 unités par cm³ d'inoculum. La contamination est réalisée par pulvérisation de l'inoculum à la surface inférieure des feuilles.

Les plants contaminés sont ensuite mis en incubation pendant sept jours à 20-22°C sous 90-100% d'humidité relative sous lumière naturelle.

La lecture se fait 7 jours après la contamination, en comparaison avec les plants témoins.

Les résultats obtenus sont reportés sous forme de points, correspondant à 50% de destruction du parasite et placés dans un diagramme d' isobole de Tammes qui comporte en abcisse les doses de A exprimées en ppm (mg/l) et en ordonnée les doses de B également en ppm (mg/l).

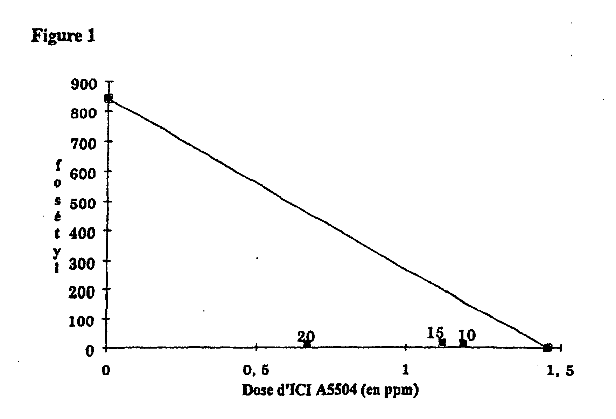

On obtient le diagramme de la figure 1. Il apparaît que l'addition de Fosétyl-Al permet, de façon parfaitement inattendue, d'abaisser la dose de A nécessaire à la destruction de 50 % du parasite en dessous de 1,5 ppm (mg/l) qui correspond à la dose de A seul qu'il est nécessaire d'appliquer pour obtenir le même pourcentage de destruction.

La disposition des points obtenue indique donc un effet bilatéral, qualifié en langue anglaise selon la méthode de Tammes citée précédemment de "two sided effect". Cette disposition correspond à une isobole de type III selon ladite méthode (page 75 de la référence bibliographique correspondante déjà citée) et est caractéristique d'une synergie.

## Revendications

1. Composition fongicide comprenant un composé A qui est le méthyl-(E)-2-{2-[6-(2-cyanophenoxy)pyrimidin-4-yloxy]phenyl}-3-methoxyacrylate,
et au moins un composé fongicide B choisi dans le groupe comprenant les dérivés de l'acide phosphoreux comme les phosphites métalliques tel que le fosétyl-Al, et l'acide phosphoreux lui-même et ses sels alcalins ou alcalino-terreux;
ladite composition comprenant les composants A et B dans un rapport en poids A/B, compris entre 0,004 et 1, de préférence entre 0,0125 et 0,4.

2. Composition fongicide selon la revendication 1 , **caractérisée en ce que** le composé B est le fosétyl Al.

3. Composition fongicide selon l'une des revendications 1 ou 2, **caractérisée en ce que** le rapport A/B est compris entre 0,01 et 1, de préférence entre 0,033 et 0,4.

4. Composition fongicide selon l'une quelconque des revendications 1 à 3, **caractérisée en ce qu'** elle comprend les composés A et B en mélange avec les supports solides ou liquides acceptables en agriculture et/ou les agents tensio-actifs également acceptables en agriculture.

5. Composition fongicide selon l'une quelconque des revendications 1 à 4, **caractérisée en ce qu'** elle comprend de 0,05 à 95 % (en poids) de matière active.

6. Procédé de contrôle des champignons phytopathogènes d'un milieu **caractérisé en ce que** l'on applique au dit milieu au moins un composé A et au moins un composé B, ces composés étant tels que définis à la revendication 1, et la combinaison de ces composés étant dans une quantité totale efficace et non phytotoxique

7. Procédé de lutte, à titre curatif ou préventif, contre les champignons phylopathogènes des cultures ou du gazon, **caractérisé en ce que** l'on applique sur les parties aériennes des végétaux une quantité efficace et non phytotoxique d'une composition fongicide selon l'une quelconque des revendications 1 à 5.

8. Procédé de lutte contre les champignons phytopathogènes des cultures selon la revendication 7, **caractérisé en ce que** l'on applique une dose comprise entre 550 et 5500 g/ha, de préférence entre 1100 et 3400 g/ha, de composition.

9. Procédé de lutte contre les champignons phytopathogènes du gazon selon la revendication 7, **caractérisé en ce que** l'on applique une dose comprise entre 5100 et 27000 g/ha, de préférence entre 10250 et 21000 g/ha, de composition.

## Patentansprüche

1. Fungizide Zusammensetzung enthaltend eine Verbindung A, bei der es sich um (E)-2-{2-[6-(2-Cyanphenoxy)pyrimidin-4-yloxy]phenyl}-3-methoxyacrylsäureester handelt,
sowie mindestens eine fungizide Verbindung B aus der Gruppe der Derivate der phosphorigen Säure, wie Metallphosphite, z.B. Fosetyl-Al, phosphorige Säure selbst sowie ihre Alkali- oder Erdalkalisalze,
wobei diese Zusammensetzung die Komponenten A und B in einem Gewichtsverhältnis A:B zwischen 0,004 und 1, vorzugsweise zwischen 0,0125 und 0,4, enthält.

2. Fungizide Zusammensetzung nach Anspruch 1, **dadurch gekennzeichnet, daß** es sich bei der Verbindung B um Fosetyl-Al handelt.

3. Fungizide Zusammensetzung nach Anspruch 1 oder 2, **dadurch gekennzeichnet, daß** das Verhältnis A:B zwischen 0,01 und 1, vorzugsweise zwischen 0,033 und 0,4, liegt.

4. Fungizide Zusammensetzung nach einem der Ansprüche 1 bis 3, **dadurch gekennzeichnet, daß** sie die Verbindungen A und B in Abmischung mit landwirtschaftlich unbedenklichen festen oder flüssigen Trägern und/oder mit ebenfalls landwirtschaftlich unbedenklichen Tensiden enthält.

5. Fungizide Zusammensetzung nach einem der Ansprüche 1 bis 4, **dadurch gekennzeichnet, daß** sie 0,05 bis 95(Gew.)-% Wirkstoff enthält.

6. Verfahren zur Bekämpfung von phytopathogenen Pilzen an einem Ort, **dadurch gekennzeichnet, daß** man mindestens eine Verbindung A und mindestens eine Verbindung B auf diesen Ort ausbringt, wobei diese Verbindungen wie in Anspruch 1 definiert sind und wobei die Kombination dieser Verbindungen in einer wirksamen, nicht pflanzenschädigen Gesamtmenge vorliegt.

7. Verfahren zur kurativen oder präventativen Bekämpfung von phytopathogenen Pilzen von Kulturpflanzen oder des Rasens, **dadurch gekennzeichnet, daß** man eine wirksame, nicht pflanzenschädigende Menge einer fungiziden Zusammensetzung nach einem der Ansprüche 1 bis 5 auf die oberirdischen Pflanzenteile ausbringt.

8. Verfahren zur Bekämpfung von phytopathogenen Pilzen von Kulturpflanzen nach Anspruch 7, **dadurch gekennzeichnet, daß** man die Zusammensetzung in einer Aufwandmenge von 500 bis 5500 g/ha, vorzugsweise 1100 bis 3400 g/ha, ausbringt.

9. Verfahren zur Bekämpfung von phytopathogenen Pilzen des Rasens nach Anspruch 7, **dadurch gekennzeichnet, daß** man die Zusammensetzung in einer Aufwandmenge von 5100 bis 27000 g/ha, vorzugsweise 10250 bis 21000 g/ha, ausbringt.

## Claims

1. Fungicidal composition comprising a compound A which is methyl (E)-2-{2-[6-(2-cyanophenoxy)-pyrimidin-4-yloxy]phenyl}-3-methoxyacrylate,
and at least one fungicidal compound B chosen from the group comprising derivatives of phosphorous acid, for example metal phosphites, such as fosetyl-Al, and phosphorous acid itself and its alkali metal or alkaline-earth metal salts;
the said composition comprising the components A and B in an A/B ratio by weight of between 0.004 and 1, preferably between 0.0125 and 0.4.

2. Fungicidal composition according to Claim 1, **characterized in that** the compound B is fosetyl-Al.

3. Fungicidal composition according to either of Claims 1 and 2, **characterized in that** the A/B ratio is between 0.01 and 1, preferably between 0.033 and 0.4.

4. Fungicidal composition according to any one of Claims 1 to 3, **characterized in that** it comprises the compounds A and B as a mixture with the solid or liquid vehicles which are acceptable in agriculture and/or the surface-active agents which are also acceptable in agriculture.

5. Fungicidal composition according to any one of Claims 1 to 4, **characterized in that** it comprises from 0.05 to 95% (by weight) of active material.

6. Process for controlling phytopathogenic fungi of a medium, **characterized in that** at least one compound A and at least one compound B are applied to the said medium, these compounds being as defined in Claim 1 and the combination of these compounds being in an effective and non-phytotoxic total amount.

7. Process for the curative or preventive control of fungi which are phytopathogenic towards crops or lawns, **characterized in that** an effective and non-phytotoxic amount of a fungicidal composition according to any one of Claims 1 to 5 is applied on the aerial parts of the plants.

8. Process for the control of fungi which are phytopathogenic towards crops according to Claim 7, **characterized in that** a dose of between 550 and 5500 g/ha, preferably between 1100 and 3400 g/ha, of composition is applied.

9. Process for the control of fungi which are phytopathogenic towards lawns according to Claim 7, **characterized in that** a dose of between 5 100 and 27 000 g/ha, preferably between 10 250 and 21 000 g/ha, of composition is applied.
